Europäisches Patentamt

European Patent Office

Office européen des brevets

(11) Publication number : **0 501 933 A2**

# EUROPEAN PATENT APPLICATION

(21) Application number : **92830087.0**

(22) Date of filing : **27.02.92**

(51) Int. Cl.$^5$ : **C07C 17/38**

PRIORITY 280292 IT MI91000526.

(30) Priority : **28.02.91**

(43) Date of publication of application :
**02.09.92 Bulletin 92/36**

(84) Designated Contracting States :
**DE FR GB NL**

(71) Applicant : **SAES GETTERS S.p.A.**
**Via Gallarate, 215/217**
**I-20123 Milano (IT)**

(72) Inventor : **Succi, Marco**
**Via Lo Monaco, 9**
**Milano (IT)**
Inventor : **Solcia, Carolina**
**Via Lazio, 7**
**Grezzano (Milano) (IT)**

(74) Representative : **Adorno, Silvano et al**
**c/o SOCIETA' ITALIANA BREVETTI S.p.A. Via**
**Carducci, 8**
**I-20123 Milano (IT)**

(54) **Process for the purification of fluorine-containing gases.**

(57)   A process is provided for the purification of an impure fluorine-containing gases containing impurities, such as $H_2O$ and $O_2$. The purification takes place by contacting the impure gas with a Zr-V-Fe alloy at less than 150°C, which has previously been activated at a temperature of greater than 300°C for longer than 10 minutes.

## Fig.1

The present invention relates to a process for the purification of an impure fluorine-containing gas, containing impurities such as $H_2O$ and $O_2$.

The dramatic scaling down of the geometries of silicon based devices is leading to the very stringent control of contaminants through the entire production cycle. The concentration of the gaseous impurities in process gases is one important contaminant.

The purity of the gas is of great importance as the yield of the circuits manufactured depends to a great degree upon this purity. While it is not possible to say what impurity level is associated with a given degree or numner of defects found in any circuit it is possible to say that the higher the purity of gas used the greater is the yield of usable circuits.

In the past it has been common practice to use organic hydrocarbons or plastics for the removal of impurities from fluorine-containing gases used for etching $SiO_2$ such as $CF_4$, $CHF_3$, $C_2F_6$, $CF_2Cl_2$ and $SF_6$. However their use has led to one or more disadvantages.

With organic hydrocarbons or plastic sorbents an increase of the operating temperature can cause the release of hydrocarbons with subsequent recontamination of the purified gas. The very nature of the materials is such that they may decompose and lead to the production of unwanted gases.

It is therefore an object of the present invention to provide an improved process for the removal of impurity gases from impure fluorine-containing gases.

It is another object of the present invention to provide an improved process for the removal of $H_2O$ and $O_2$ from fluorine-containing gases, which have $H_2O$ and $O_2$ impurities.

It is yet another object of the present invention to provide an improved process which does not require the use of organic support or plastics for the removal of impurities from fluorine-containing gases.

These and other advantages and objects of the present invention will become clear to those skilled in the art by reference to the following description and the drawings in which:

Fig. 1 is a schematic diagram, partially cut away, of an apparatus useful in a process of the present invention; and

Fig. 2 is a graph showing decomposition curves of fluorine-containing gases over the getter alloy used in the present invention.

The invention provides a process for the removal of impurity gases from impure fluorine-containing gases. It comprises the steps of heating an alloy of Zr-V-Fe to a temperature of greater than 300°C for a time of greater than 10 minutes. The alloy is then cooled to a temperature below which there is no appreciable dissociation of the fluorine-containing gas. At this temperature the impure fluorine-containing gas is brought into contact with the Zr-V-Fe alloy.

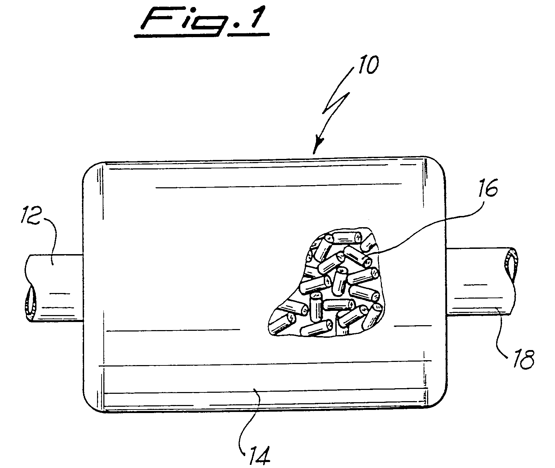

With reference to Fig. 1 there is illustrated an apparatus 10 useful in carrying out the process of the present invention for the removal of impurity gases from impure fluorine-containing gases. Apparatus 10 comprises an impure gas inlet 12 through which enters the impure fluorine-containing gas. Inlet 12 is in fluid communication with a chamber 14 for the purification of the fluorine-containing gas and contains a getter alloy 16. Chamber 14 is also in fluid communication with a purified gas outlet 18.

Getter alloy 16 is in the form of a powder of particle size preferably less than 500μm and preferably less than 125μm. It is preferably in the form of pellets, with or without a binder, suitable for incorporation within chamber 14.

The composition of the alloy is Zr-V-Fe.

Typical compositions of the ternary alloy according to the invention comprise :

    Zr from 45 to 75, and preferably from 47 to 70% by weight

    V from 20 to 50, and preferably from 24 to 45% by weight

    Fe from 5 to 35, and preferably from 5 to 10% by weight.

the compositions in weight percent when plotted on a ternary composition diagram in weight percent Zr, weight percent V and weight percent Fe lies within a polygon having as its corners the points defined by:

    (a) 75% Zr - 20% V - 5% Fe

    (b) 45% Zr - 20% V - 35% Fe

    (c) 45% Zr - 50% V - 5% Fe.

and preferably lies within the polygon having as its corners the points defined by:

    (d) 70% Zr - 25% V - 5% Fe

    (e) 70% Zr - 24% V - 6% Fe

    (f) 66% Zr - 24% V - 10% Fe

    (g) 47% Zr - 43% V - 10% Fe

    (h) 47% Zr - 45% V - 8% Fe

    (i) 50% Zr - 45% V - 5% Fe.

Alloys useful in the present invention are described in Boffito et al U.S. patent 4,312,669.

It should be realized that small amounts of other metals can be used without substantially altering its purification characteristics. For instance the Fe may be partially replaced by Ni, or the V may be partially replaced with Nb. It may be advantageous to replace some of the Zr with Ti without substantially altering the main sorption ability of the basic ternary alloy. One or more substitutions may take place at the same time.

The temperature of activation of the Zr-V-Fe alloy should be high enough to clean the surface of the particles form oxides and nitrides that have formed during air exposure. This can be accomplished by heating in a vacuum or a flow of rare or inert gas to a temperature of greater than 300°C for a time of longer than 10 minutes, for example 400°C for 4 hours.

The alloy, when sorbing impurities from fluorine-containing gases must not cause its dissociation and must be held at a temperature below the temperature of appreciable dissociation of fluorine-containing gas. This temperature should be less than 150°C and is preferably 100°C. A temperature of 100°C is sufficiently low to prevent fluorine-containing gas dissociation but sufficiently high to cause the Zr-V-Fe alloy to be active towards impurity gases.

The invention may be better understood by reference to the following examples wherein all parts and percentages are by weight unless otherwise indicated. These examples are designed to each those skilled in the art how to practice the result invention and represent the best mode presently known for practicing the invention.

EXAMPLE 1

This example is given to determine the temperatures above which the fluorine-containing gases start to dissociate or decompose.

A chamber 14 of a device 10 is filled with 70 g of pellets 4 mm in diameter and 3 mm height of compressed Zr-V-Fe powder. The nominal composition of the powder was 70% Zr - 24.6% V - 5.4% Fe by weight.

Argon, containing 300 parts per million (ppm) of fluorine-containing gas, was caused to flow at 6.05 l/min.

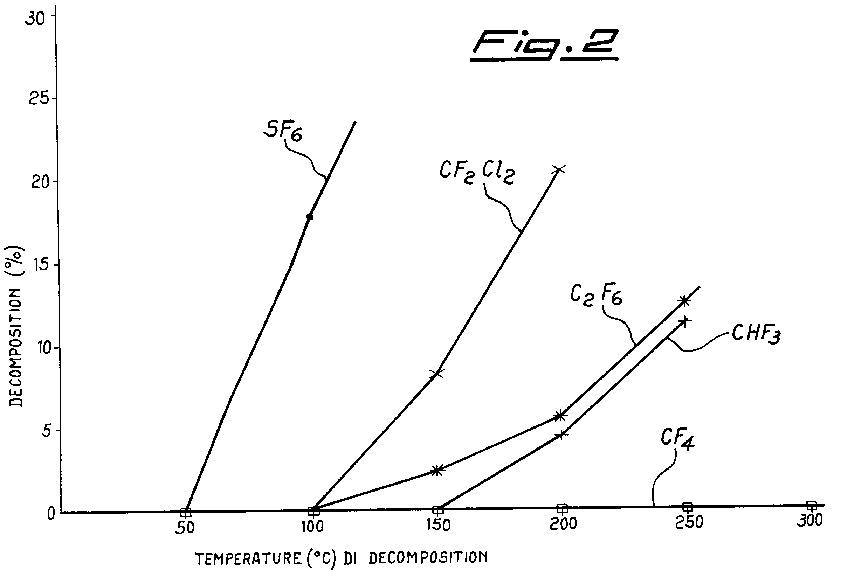

The temperature of the purifier was progressively increased from room temperature up to the decomposition temperature of the fluorine-containing gases in steps of 50°C. Figure 2 shows the results obtained for $CF_4$, $CHF_3$, $C_2F_6$, $CF_2Cl_2$ and $SF_6$ using a VARIAN 3400 gas chromatograph and the decomposition temperatures $T_D$ are reported in the table below:

| Fluorine-containing gases | $T_D$ |
|---|---|
| $CF_4$ | $> 150°C$ |
| $CHF_3$ | $150°C$ |
| $CF_2Cl_2$, $C_2F_6$ | $100°C$ |
| $SF_6$ | $50°C$ |

EXAMPLE 2

The efficiency of removal of impurities such as oxygen and water vapour was evaluated by means of the apparatus shown in fig. 1. A purifier containing a small quantity of getter material (70 g) as in Example 1 was activaved in argon at 400°C for 3 hours in order to remove the thin oxide film that passivates the surface. Then the temperature was reduced down to 100° and the purifier was ready to work with fluorine-contairing gases. $CHF_3$ containing 17.5 ppm of oxygen and 9.2 ppm of moisture was passed through the purifier. The concentration of the impurities was measured by the oxygen analyzer MKIII/Y made by OSAKA OXYGEN and by the moisture analyzer AQUAMATIC+ made by MEECO. The former is based on an electrolytic cell, the latter on the decomposition of the moisture trapped by an hygroscopic film; the sensitivities are respectively 2 ppb and 20 ppb. Although the size of the purifier was small and the concentration of the impurities at the inlet was high, both analyzers indicated a value below the sensitivity, showing an extremely good removal of the impurities.

EXAMPLE 3

Due to the problem of controlling the impurities in fluorine-containing gases, the capacity test was carried out in argon. Since at 100°C there is no decomposition of the fluorine-containing gases on the getter, it was assumed that the capacity obtained in this condition is the same that would have been obtained flowing fluorine-containing gases.

To limit the duration of the test, purifier containing only a small amount of getter material has been used: 20 g. It was activated at 400°C for 3 hours; then the temperature was set at 100°C and argon containing 14.5 ppm of oxygen and about 11 ppm of moisture was passed through it. The concentration of oxygen and water vapour was continuously monitored with the same analyzers.

Capacity is the quantity of impurities sorbed by the purifier before measuring any increase of the impurities at the outlet of the purifier over the mass of getter or the volume of the purifier.

Flowing 0.3 l/min., the breakthrough of the impurities was detected after 7 days of continuous operation. Both gases started increasing at the outlet of the purifier at the same time showing that the active sites for the two gases are the same. This means that the available capacity is shared between the two gases and thus the higher the sorption for oxygen, the lower is the sorption for water vapour and vice versa. The capacity is 1.6 torr 1 of oxygen per g of getter material and 1.2 torr 1 of moisture per g of getter material (or 6.2 STP 1/1 for oxygen and 5 STP 1/1 for moisture).

The difference between the capacity for oxygen and water vapour is probably due to the higher oxygen concentration in the inlet gas.

Although the invention has been descrinbed in considerable detail with reference to certain prefered embodiments designed to teach those skilled in the art how best to practice the invention, it will be realized that other modifications may be employed without departing from the spirit and scope of the appended claims.

## Claims

1. A process for the removal of impurity gases from impure fluorine-containing gas which comprises the steps of;

   A. heating an alloy of Zr-V-Fe to a temperature of greater than 300°C for a time of greater than 10 minutes;

   B. cooling the alloy to a temperature below the temperature of appreciable dissociation of the fluorine-containing gas; and

   C. contacting the impure fluorine-containing ,as with the Zr-V-Fe alloy.

2. A process for the removal of impurity gases from impure fluorine-containing gas by passing the impure fluorine-containing gas through a chamber having an impure gas inlet, and a purified gas outlet, said chamber containing a Zr-V-Fe alloy, said process comprising the steps of:

   A. heating said alloy of Zr-V-Fe in a flow or rare or inert gas to a temperature of greater than 300°C for a time of greater than 10 minutes to cause it to become gas sorbing;

   B. cooling the alloy to a temperature of less than 150°C whereby it does not cause appreciable dissociation of the fluorine-containing gas; and

   C. containing the impure fluorine-containing gas with the Zr-V-Fe alloy.

3. A process for the removal of $H_2O$ and $O_2$ from impure fluorine-containing gas by passing the $H_2O$ and $O_2$ in the fluorine-containing gas through a chamber having an impure gas inlet, and a purified gas outlet, said chamber containing a Zr-V-Fe alloy, said process comprising the steps of

   A. heating said alloy of Zr-V-Fe in a flow of inert or rare gas to a temperature of greater than 300°C for a time of greater than 10 minutes to cause it to become gas sorbing;

   B. cooling the alloy to a temperature of less than 150°C whereby it does not cause appreciable dissociation of the fluorine-containing gas; and

   C. containing the $H_2O$ and $O_2$ of the impure fluorine-containing gas with the Zr-V-Fe alloy whereby the $H_2O$ and $O_2$ are removed stoichiometrically together with the $O_2$.

4. The process of Claim 1 wherein the fluorine-containing gas is $CF_4$.

5. The process of Claim 1 wherein the fluorine-containing gas is $CHF_3$.

6. The process of Claim 1 wherein the fluorine-containing gas is $CF_2Cl_2$.

7. The process of Claim 1 wherein the fluorine-containing gas is $C_2F_6$.

4

8. The process of Claim 1 wherein the fluorine-containing gas is $SF_6$.

9. The process of Claim 1 wherein the fluorine-containing gas is a fluorine etching gas.

# Fig.1

*Fig.2*

DECOMPOSITION (%)

TEMPERATURE (°C) DI DECOMPOSITION

SF$_6$

CF$_2$ Cl$_2$

C$_2$ F$_6$

CHF$_3$

CF$_4$

EP 0 501 933 A2